# EUROPEAN PATENT APPLICATION

(11) **EP 3 026 601 A1**
(43) Date of publication of application: **01.06.2016**
(21) Application number: 14195151.7
(22) Date of filing: 27.11.2014
(51) Int. Cl.: G06N 5/04

(54) **Data repository querying method**

(71) Applicant: Agfa Healthcare, 2640 Mortsel (BE)
(72) Inventor: Depraetere, Kristof, 2640 Mortsel (BE); Sun, Hong, 2640 Mortsel (BE); De Roo, Jos, 2640 Mortsel (BE); Colaert, Dirk, 2640 Mortsel (BE)
(74) Representative: Verbrugghe, Anne Marie L.

(57) **Abstract**

In a computer-implemented method of querying a data repository wherein a software image is generated of a at least one of a pre-compiled semantic reasoning engine, a set of pre-compiled fact data and pre-compiled reasoning rules. At runtime additional run time data and / or run time reasoning rules are loaded. A query is specified and solved by means of the software image and the additional run time data and / or runtime rules.

## Description

### FIELD OF THE INVENTION

The present invention relates to rule based reasoning. The invention more specifically relates to a computer-implemented method for querying a data repository, e.g. a semantic data repository, and deriving a response to that query by means of rule based reasoning. The invention is inter alia applicable in the field of clinical decision support.

### BACKGROUND OF THE INVENTION

Clinical decision support is a technique to help physicians with decision making tasks, such as obtaining a diagnosis for a patient.

Clinical decision support systems generally execute queries on large data repositories of patient data. Clinical terminology is used in such queries in expressing the domain of interest.
Rule based reasoning is applied to execute queries on such data repositories.

Executing queries on a data repository by means of state of the art rule based reasoning techniques may take a large amount of computational effort, which might be unacceptable.

There may be a need to use large rule files, e.g. in case of specialization / materialization, or large fact files, e.g. in case of terminologies used by a reasoning engine to solve a problem.

Loading such large files in a reasoning engine can take a substantial amount of time, e.g. several seconds may be needed for SNOMED CT (SNOMED CT being a well-known systematically organized computer processable collection of medical terms providing codes, terms, synonyms and definitions used in clinical documentation and reporting).

It is therefore an aspect of the present invention to provide a technique that results in a decrease of the required computational effort.

### SUMMARY OF THE INVENTION

The above-mentioned aspect is obtained by a computer-implemented method of querying a data repository having the specific method steps set out in claim 1.

Specific features for preferred embodiments of the invention are set out in the dependent claims.

According to the present invention a reasoning engine, a set of reasoning rules as well of a set of data relating to facts (fact statements) are compiled into a software image, which is a binary, reasoner specific representation of the reasoning engine, the rules and the fact data.

This representation is advantageous since the binary image representation can be loaded much faster than a textual form of the rules.

The software image can be pre-compiled at development time. Alternatively it can be compiled at deployment, e.g. when a first time request comes in.

In the context of the present invention the term 'pre-compiled' refers to the translation of rules, fact data and reasoning engine from its textual representation, e.g. source code, into byte code, e.g. into Prolog virtual machine instructions.

Fact data are commonly represented in one of N3, Turtle, RDF ...

Rules can be specialized or materialized as set out in co-pending European patent application 14157487.
As has been disclosed in this application, starting from a set of generic reasoning rules a set of specific reasoning rules is generated by substituting in at least one of said generic reasoning rules having more than one variable at least one of said variables with (a) class(es) defined in an ontology.
Such specialized or materialized rules can be pre-compiled in a software image.

The selection of rules and data that are pre-compiled into the software image depends on the use case (e.g. regarding a SNOMED class).

An image is created comprising a dedicated reasoning engine and predetermined rule files and/or fact files.
Because the image contains a predefined set of rules and/or facts it effectively becomes a dedicated reasoning engine targeted to deduce additional facts based on the embedded rules and /or facts.

Additional rules and/or facts can be added at runtime to further extend the dedicated reasoning engine, but the predefined rules and/or facts can not be removed from the dedicated reasoning engine.

In one embodiment the dedicated reasoning engine according to the present invention can be embedded into the configuration of a web resource service.
When the URL of the web resource is resolved, the service executes the dedicated reasoning image on the file system.
The supplied URL (query) parameters are provided to the dedicated reasoning image and the additionally provided rules and / or facts. The supplied (query) parameters are also used to specify the semantic query on the reasoning result.
The result of the query is serialized and transferred to the client.
Alternatives are a command line, a Java script ...

In the context of the present invention a semantic reasoning engine is envisaged which is commonly defined as software able to infer logical consequences from a set of asserted facts or axioms. In a semantic reasoning engine, rules are specified by means of an ontology language and often a description language. (Source : Wikipaedia).
In the context of the present invention this reasoning engine is the EYE reasoner.

The method of the present invention is generally implemented in the form of a computer program product adapted to carry out the method steps of the present invention when run on a computer. The computer program product is commonly stored in a computer readable carrier medium such as a DVD. Alternatively the computer program product takes the form of an electric signal and can be communicated to a user through electronic communication.

The present invention has been developed with the aim of optimizing the querying of data repositories of clinical patient information in a healthcare environment. The field of application of the present invention is however not limited thereto.

The invention can be used in other applications based on rule-based reasoning as well as on data representing other types of information than clinical information.

Examples of applications in which the invention can be used:
Patient data (e.g. data out of a patient record) may be made available in the image. A query can be performed which requires decision support for these specific patient data.

Population data may be available in the image or may be added at runtime to perform the stored analysis. Additional rules may be applied for population analysis.

In still another example the image comprises a dedicated hospital formularium comprising only the specific data on medical treatment used by the hospital.

In still another example the image may comprise precompiled medical knowledge for a specific medical sub domain for triage in emergency department, e.g. for headache. Patient data can be supplied at runtime.

The invention may also be used for example in the context of an alert system for e.g. a dentist wherein a pre-defined image according to the present invention is combined with data regarding a patient at run-time.

## Claims

1. A computer-implemented method of querying a data repository comprising the steps of
- executing a software image comprising at least one of a pre-compiled reasoning engine, a set of pre-compiled fact data and a pre-compiled reasoning rules,
- loading additional run time data and / or run time reasoning rules,
- loading a query,
- solving said query by means of said image and said additional run time data and / or runtime rules,
- providing a query result.

2. A method according to claim 1 wherein said software image is generated pre-run time.

3. A method according to claim 1 wherein said software image is generated at run time.

4. A method according to claim 1 wherein said image is used by a HTTP resource.

5. A method according to claim 1 wherein said result is returned as an HTTP response.

6. A method according to claim 1 wherein the rules and/or facts are semantic web rules and/or data.

7. A method according to claim 1 wherein said rules are expressed with N3.

8. A method according to claim 1 wherein said facts are expressed with RDF.

9. A method according to claim 1 wherein said rules and /or facts are translated into prolog virtual machine byte code.

10. A method according to claim 1 wherein said reasoning engine is EYE.

11. A computer program product adapted to carry out the method of any of the preceding claims when loaded into a computer.

12. A computer readable medium comprising computer executable program code adapted to carry out the steps of any of claims 1 - 10.
